(19) Europäisches Patentamt / European Patent Office / Office européen des brevets

(11) EP 4 439 063 A1

(12) **EUROPEAN PATENT APPLICATION**
published in accordance with Art. 153(4) EPC

(43) Date of publication:
02.10.2024 Bulletin 2024/40

(21) Application number: 22898750.9

(22) Date of filing: 19.07.2022

(51) International Patent Classification (IPC):
*G01N 33/50* (2006.01)

(52) Cooperative Patent Classification (CPC):
C12Q 1/02; C12N 5/0693; C12N 13/00;
G01N 33/50; G01N 33/5011

(86) International application number:
PCT/KR2022/010503

(87) International publication number:
WO 2023/096065 (01.06.2023 Gazette 2023/22)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 24.11.2021 KR 20210163284
18.07.2022 KR 20220088270

(71) Applicants:
• MBD Co., Ltd.
Suwon-si, Gyeonggi-do 16229 (KR)

• Samsung Life Public Welfare Foundation
Seoul 04348 (KR)

(72) Inventors:
• KU, Bosung
Yongin-si, Gyeonggi-do 16939 (KR)
• KIM, Jungeun
Yongin-si, Gyeonggi-do 16813 (KR)
• CHUNG, Manki
Seoul 06360 (KR)
• LEE, Dongwoo
Daejeon 35381 (KR)

(74) Representative: Gulde & Partner
Patent- und Rechtsanwaltskanzlei mbB
Wallstraße 58/59
10179 Berlin (DE)

(54) **METHOD FOR DIAGNOSING RADIATION SENSITIVITY**

(57) Disclosed herein is a radiation sensitivity diagnosis method using a radiation sensitivity diagnosis kit including a radiation irradiating device and a control unit, the method including the steps of: (a) dispensing one or more cancer cells separated from an individual on a CRS plate of the diagnosis kit; (b) culturing the at least one cancer cell to form one or more cancer organoids by filling the CRS plate with a culture medium or immersing the CRS plate in a well; (c) irradiating radiation rays, which passed a radiation dose control unit, to the CRS plate from the radiation generating unit of the radiation irradiating device; (d) measuring cell viability of the at least one cancer organoid; (e) obtaining cell experiment-based data on the basis of the measured cell viability; and (f) diagnosing radiation sensitivity for the individual by the control unit on the basis of the cell experiment-based data.

The radiation sensitivity diagnosis method can effectively reduce the cost and time for radiation sensitivity diagnosis by irradiating different radiation doses to a plurality of cancer organoids disposed in a radiation sensitivity diagnosis kit, and can accurately predict radiation therapy effect by determining radiation sensitivity using cell experiment-based data derived from individual patients, thereby providing information necessary for determining whether to perform radiation therapy for preservative treatment.

FIG. 1

| | |
|---|---|
| Dispense cancer cells separated from an individual on a CRS plate of a diagnosis kit | S111 |
| Culture the cancer cells by filling the CRS plate with a culture medium to form organoids | S112 |
| Irradiate radiation rays by a radiation dose control device of the diagnosis kit | S120 |
| Measure cell viability of the cancer organoids to which radiation rays are irradiated | S130 |
| Calculate a cell growth factor and a radiation response factor of the cancer organoids to which radiation rays are irradiated | S140 |
| Diagnose radiation sensitivity using a radiation sensitivity prediction model | S150 |

EP 4 439 063 A1

## Description

### Technical Field

The present invention relates to a radiation sensitivity diagnosis method, and more specifically, to a radiation sensitivity diagnosis method for predicting and diagnosing radiation sensitivity and prognosis of a patient before radiation therapy by using a radiation sensitivity diagnosis kit in which cancer tissue coming from a lesion tissue of a patient is cultivated in an extracorporeal environment similar to that of a living body, and radiation rays of various radiation doses are irradiated to the cultured cancer cells to diagnose radiation sensitivity.

### Background Art

Usually, treatment of cancer can include chemotherapy, radiation therapy, and a variety of drug therapies including antibody-based drugs depending on kinds of cancers. Among these therapies, the radiation therapy is performed for carcinomas that is surgically inoperable or is deteriorated in efficiency of anticancer drugs, and is performed for the purpose of maintaining the high quality of life and prolonging the life by removing cancer cells while minimizing a damage of normal tissues rather than perfectly eliminating cancer cells, the ultimate causes.

Therefore, radiation therapy is carried out for the purpose of conservative treatment, which accurately administers the planned radiation dose to the target cancer, treats the symptoms, and expects improvement of the disease. Through this treatment, effects such as removal or size reduction of the lesion tissue before surgery, prevention of recurrence after cancer treatment in other methods, and relief of nerve/organ pressure due to an increase in the size of cancer cells can be achieved. In particular, a conservative treatment strategy using radiation therapy is recognized as an effective treatment method for head and neck cancer, gastrointestinal cancer, urinary cancer, esophageal cancer, brain tumor, lung cancer, liver cancer, rectal cancer, anal cancer, cervical cancer, etc.

To achieve the goal of the conservative treatment, a method for accurately diagnosing and predicting an individual patient's response to the radiation therapy is very important. In other words, an approach to which the concept of precision medicine is applied is essential, which predicts the radiation therapy response of individual patients and applies them to treatment. However, although there have been many studies to find factors that predict radiation sensitivity (imaging indicators, genomic indicators, etc.), they have not been applied to clinical practice and currently, it is dependent on the experience of the clinician.

Especially, although radiation sensitivity prediction using tissue from a cancer patient requiring conservative treatment is necessary, a radiation therapy reaction prediction study using the 3D cultivation of patient-derived cells is not yet attempted, even a study to compare a radiation sensitivity prediction test result with a clinical result and confirm a coincidence level has not been made up to date.

### Disclosure

### Technical Problem

Accordingly, the present invention has been made in view of the above-mentioned problems occurring in the related art, and it is an object of the present invention to provide a radiation sensitivity diagnosis method capable of accurately determining radiation sensitivity of individual patients with the most effective factors among various factors previously studied by considering radiation response factors and cell growth factors at the same time.

It is another object of the present invention to provide a radiation sensitivity diagnosis method capable of effectively reducing the cost and time for radiation sensitivity diagnosis by irradiating different radiation doses to each of a plurality of cancer organoids disposed in a radiation sensitivity diagnosis kit.

In addition, it is a further object of the present invention to provide a radiation sensitivity diagnosis method capable of accurately predicting radiation therapy effect by determining radiation sensitivity with cell experiment-based data derived from individual patients, thereby providing information necessary for determining whether to perform radiation therapy for preservative treatment.

The objects of the present invention are not limited to the foregoing, and other various objects described in the detailed description of the present invention are all included in the objects of the present invention.

### Technical Solution

To accomplish the aforementioned objects, according to the present invention, there is provided a radiation sensitivity diagnosis method using a radiation sensitivity diagnosis kit including a radiation irradiating device and a control unit, the method comprising: (a) dispensing one or more cancer cells separated from an individual on a CRS plate of the diagnosis kit; (b) culturing the at least one cancer cell to form one or more cancer organoids by filling the CRS plate with a culture medium or immersing the CRS plate in a well filled with culture media; (c) irradiating radiation rays, which passed a radiation dose control unit, to the CRS plate from a radiation generating unit of the radiation irradiating device; (d) measuring cell viability of the at least one cancer organoid; (e) obtaining cell experiment-based data based on the measured cell viability; and (f) diagnosing radiation sensitivity for the individual by the control unit based on the cell experiment-based data.

Accordingly, the radiation sensitivity diagnosis method can effectively reduce the cost and time for diagnosis by irradiating different total radiation doses to a plurality of cancer organoids disposed in a radiation sensitivity diagnosis kit during the same amount of time, and can accurately predict radiation therapy effect by determining radiation sensitivity with cell experiment-based data derived from individual patients, thereby providing information necessary for determining whether to perform radiation therapy for preservative treatment.

Other details of embodiments will be included in the detailed description and drawings of the present invention.

**Advantageous Effects**

Therefore, according to the present invention, the radiation sensitivity diagnosis method can effectively diagnose a patient's radiation sensitivity to cancer cells to predict effects of radiation therapy. Therefore, the radiation sensitivity diagnosis method according to the present invention allows a user to accurately and effectively determine whether to perform radiation therapy with respect to a cancer suitable for the preservation treatment, such as head and neck cancer, lung cancer, esophagus cancer, rectal cancer, anal cancer, cervical cancer, etc.

Moreover, the radiation sensitivity diagnosis method according to the present invention can accurately determine radiation sensitivity of individual patients with the most effective factors among various factors previously studied by considering radiation response factors and cell growth factors at the same time.

In addition, according to the present invention, the limitations such as limited time and a small number of samples can be overcome and the data necessary for diagnosing radiation sensitivity can be obtained through a characteristic sensitivity diagnostic kit in which each of a plurality of cancer organoids is irradiated with different total radiation doses due to a change in relative locations of the plate unit and the radiation shield.

The effects according to the present invention are not limited by the teachings illustrated above, and more various effects are included in this specification.

**Description of Drawings**

FIG. 1 is a flow chart of a radiation sensitivity diagnosis method according to an embodiment of the present invention.
FIG. 2 is a diagram illustrating a radiation sensitivity diagnosis kit according to an embodiment of the present invention.
FIGS. 3A and 3B are views depicting a radiation sensitivity diagnosis method according to an embodiment of the present invention.
FIGS. 4 to 7 are views illustrating a radiation dose control unit of a radiation sensitivity diagnosis kit according to various embodiments of the present invention.
FIG. 8 is a diagram depicting cell viability measurement of radiation irradiated cancer organoids to which radiation is irradiated according to an embodiment of the present invention.
FIGS. 9 and 10 are diagrams illustrating radiation response factors according to an embodiment of the present invention.
FIG. 11 is a diagram illustrating a radiation sensitivity diagnosis module according to an embodiment of the present invention.
FIGS. 12 and 13 are views depicting a radiation sensitivity diagnosis method based on a radiation response factor and a cell growth factor according to an embodiment of the present invention.
FIGS. 14A and 14B are diagrams illustrating a radiation sensitivity index according to an embodiment of the present invention.
FIGS. 15A and 15B show diagnostic results of a radiation sensitivity diagnosis kit according to an embodiment of the present invention.
FIG. 16 is a view illustrating a radiation sensitivity diagnosis system according to an embodiment of the present invention.

**Best Mode**

Various embodiments of the present invention will be described in detail with reference to the accompanying drawings. Advantages and features of the present disclosure and methods accomplishing the advantages and features will become apparent from the following detailed description of exemplary embodiments with reference to the accompanying drawings. However, the present invention is not limited to exemplary embodiment disclosed herein but will be implemented in various forms. The exemplary embodiments are provided so that the present invention is completely disclosed, and a person of ordinary skilled in the art can fully understand the scope of the present invention. Therefore, the present invention will be defined only by the scope of the appended claims.

The shape, size, ratio, angle, number, and the like, which are disclosed in the drawings for depicting an embodiment of the present invention, are exemplary, and the present invention is not limited thereto. Like reference numerals refer to like elements throughout the specification. Moreover, a detailed description of known techniques related to the present invention will be omitted when it is determined that the detailed description may obscure the subject matter of the present invention. In the present specification, it should be understood that the terms of 'include' or 'have' in the specification are used to mean that there is no intent to exclude existence or addition of other components besides components described in the specification. The terms of a singular form may include plural forms unless otherwise specified.

In interpreting the components, unless otherwise defined, it is construed as including an error range.

In the description of the positional relationship, for example, in the description of the positional relationship of two parts by using the terms, such as "above", "upper", "below", "besides", and the like, it may mean that one or more other parts may be disposed between the two parts unless the term, 'directly' or 'just', is used.

It will be understood that, although the terms, first, second, etc., may be used herein to describe various elements, these elements should not be limited by these terms. These terms are used to distinguish one element from another. Thus, a first element discussed below could be termed a second element without departing from the teachings of the inventive concepts.

In the detailed description, the same reference numbers of the drawings refer to the same or equivalent parts of the present invention.

In the drawings, thicknesses and sizes of components are illustrated for convenience in explanation, and the present invention is not limited thereto.

It should be understood that all of the conditional terms and embodiments listed herein are, in principle, expressly intended for purposes of understanding the concepts of the invention, and are not particularly limited to the embodiments and states that are specifically listed.

In addition, in the following description, the mathematical expressions such as the first, second, etc. are equivalent to each other and are intended to describe independent objects, and it is to be understood that the order of the terms of main/sub or master/slave is meaningless.

The above-described objects, features, and advantages will become more apparent through the following detailed description with respect to the accompanying drawings, and thus, those skilled in the art could readily implement the technical idea of the present invention.

The features of the various embodiments of the present invention may be partially or entirely coupled or combined with one another, as will be appreciated by those skilled in the art, and as will be appreciated by those skilled in the art, the embodiments may be implemented independently of each other and may be implemented together in an associated relationship.

First, the main terms used herein are described prior to describing the present invention.

The individual may mean all objects to diagnose radiation sensitivity. For example, the individual may be who has at least one of ovarian cancer, breast cancer, squamous cell carcinoma, uterine cancer, cervical cancer, prostate cancer, head neck cancer, pancreatic cancer, brain tumor, liver cancer, skin cancer, esophageal cancer, testicular cancer, kidney cancer, colorectal cancer, rectal cancer, gastric cancer, renal cancer, bladder cancer, cholangiocarcinoma, and gallbladder cancer. Preferably, the individual may be who has head and neck cancer but is not limited thereto.

Radiation sensitivity may mean sensitivity to radiation and may be a measure to diagnose whether or not to exhibit a therapeutic response upon irradiation of radiation rays. The radiation sensitivity can be used interchangeably with response of radiation therapy, radiation response, etc. Thus, depending on the diagnosis of radiation sensitivity, the individual may be determined as a therapeutic response positive individual or a therapeutic response negative individual to radiation therapy. In addition, an individual having a relatively high sensitivity to radiation can be determined as a therapeutic response positive individual, and an individual having a relatively low sensitivity to radiation can be determined as a therapeutic response negative individual.

Meanwhile, an embodiment of the present invention relates to a cancer organoid-based radiation sensitivity diagnosis. The cancer organoid-based radiation sensitivity diagnosis measures a radiation response of the organoids by dispensing the tissue cells and the extracellular matrix of a cancer patient onto a culture plate and irradiating radiation with various doses, and the result can be utilized as a critical supplementary data in determination of radiation therapy for the patient.

In a human body, cancer cells make up a microenvironment surrounded by an extracellular matrix, and a model for predicting radiation therapy requires a biometric model capable of imitating it. Therefore, according to an embodiment of the present invention, an extracellular matrix is formed on a pillar chip, and cancer cells are cultured in the extracellular matrix to implement a microenvironment, and radiation rays are transmitted to measure the viability of cancer patient-derived cells, thereby measuring radiation sensitivity. In the present embodiment, an experiment on head and neck cancer has been described, but as mentioned above, cancer patient-derived cells, such as, for example, digestive tract cancer, urinary bladder cancer, esophageal cancer, brain tumor, lung cancer, liver cancer, rectal cancer, anal cancer, cervical cancer, and the like, which are suitable for conservative treatment, are all applicable to the present invention.

In order to measure a response to radiation rays of cancer patient-derived cells, various radiation doses must be irradiated to cancer organoids on a CRS plate. Therefore, a diagnosis kit according to an embodiment of the present invention can measure viability of the cancer patient-derived cells, for example, head and neck cancer, depending on various radiation doses on a single plate using a radiation dose control unit capable of selectively shielding or controlling the radiation doses.

According to the present invention, a radiation sensitivity diagnosis kit capable of irradiating various radiation doses to cells through one radiation irradiation in a manner to freely control the radiation doses in one diagnostic kit has been developed and used for diagnosis.

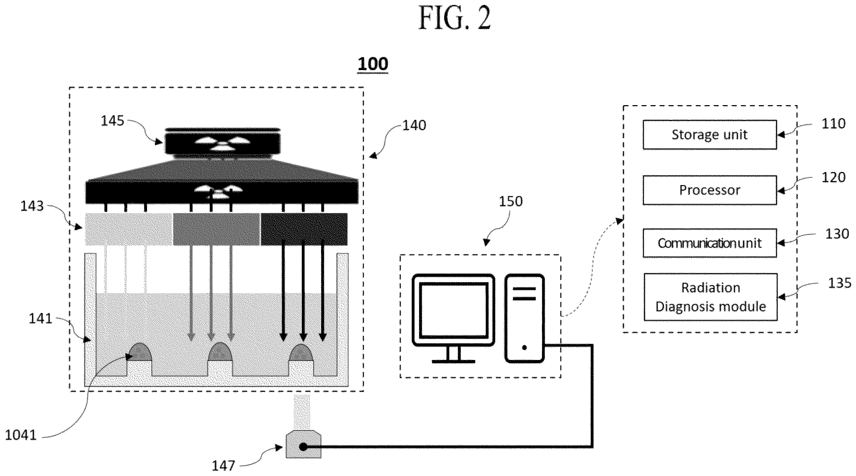

Hereinafter, a radiation sensitivity diagnosis kit 100 according to an embodiment of the present invention will be described with reference to FIGS. 2 to 7.

Referring to FIG. 2, the radiation sensitivity diagnosis kit 100 includes a radiation irradiating device 140 and a control unit 150. The control unit 150 includes a storage unit 110, a processor 120, a communication unit 130, and a radiation diagnosis module 135. The radiation sensitivity diagnosis kit 100 further includes an imaging device 147 for checking cancer organoid images, such as a fluorescent microscope.

In this instance, the radiation irradiating device 140 includes, for example, a radiation generating unit 145, a radiation dose control unit 143, and a CRS (Cell Ratio-Sensitivity) plate 141.

Specifically, the radiation generating unit 145 is a device for accelerating charged particles to generate radiation rays, and the generated radiation rays are irradiated at different radiation doses to at least a portion of one or more cancer organoids 1041 on the CRS plate 141 via the radiation dose control unit 143.

In order to irradiate radiation rays at various radiation doses depending on each cancer organoids 1041, the radiation dose control unit 143 includes any one among a slit type radiation dose modulator 243 for controlling the radiation doses through a slit 243-1 relatively moving with respect to the CRS plate 141, a shield movable type radiation dose modulator 443 for controlling radiation irradiation time for each cancer organoid by moving a shield in a rectilinear motion, a density-adjustable radiation shield 343 configured to selectively adjust the radiation doses according to the radiation transmittance controlled by the pattern, color, material (metal), material density, and the like of a radiation transmitting shield plate, and a laminated radiation shield 543 varying the number of radiation protective shields according to the radiation doses to be irradiated.

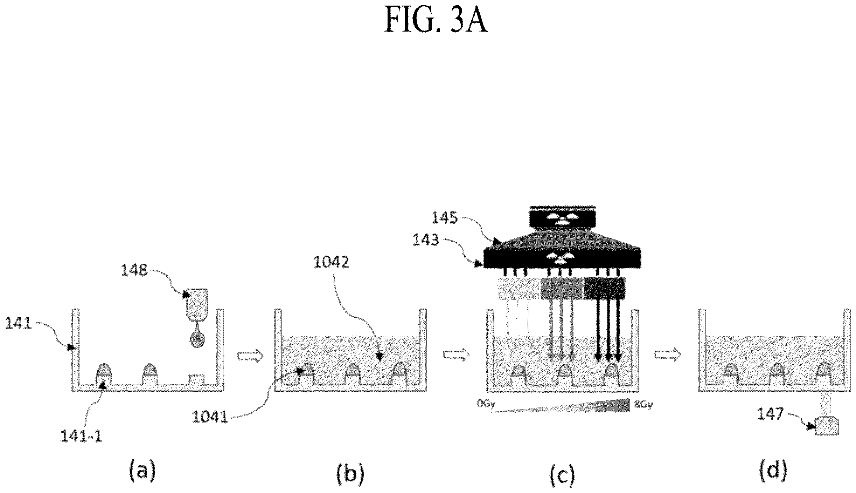

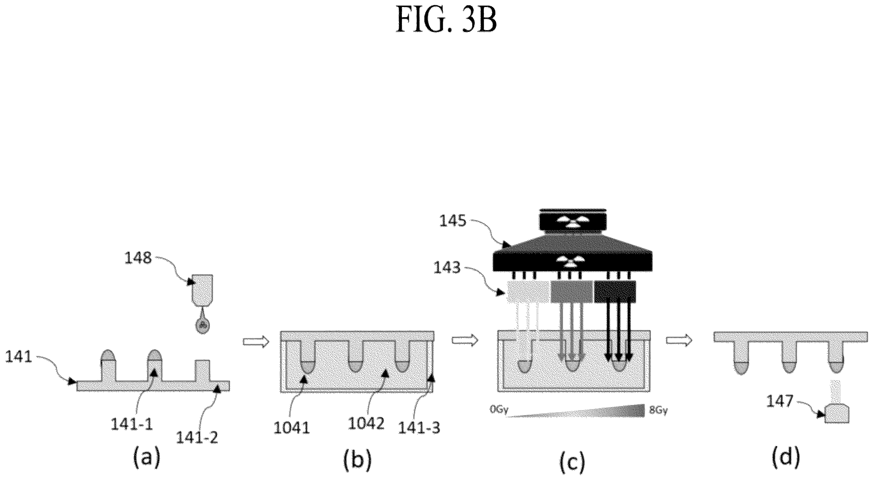

Hereinafter, the CRS plate 141 will be described in detail with reference to FIG. 3. The CRS plate 141 includes a pillar capable of dispensing cells, is capable of cell culture, and is necessary to check a radiation response by a dyeing reagent after culturing. The CRS plate 141 may have various shapes as illustrated in FIGS. 3A and 3B. In case of FIG. 3A, the CRS plate 141 includes a well which is a space receiving a culture medium 1042. And a plurality of pillar portions 141-1 protrudes in a pillar shape from the bottom surface of the receiving space in the upward direction (toward the radiation generating unit 145) so that cancer organoids can be generated by cancer cells that is cultivation objects thereon. In case of FIG. 3B, the plurality of pillar portions 141-1 are accommodated in a well 141-3 in such a way as to protrude from a bottom portion 141-2 of the CRS plate 141 in the downward direction opposite to the direction toward the radiation generating unit 145 and are immersed in the culture medium 1042. However, it should be understood by those skilled in the art that the present invention is not limited to this, and any structure is possible if the cancer cells dispensed onto the CRS plate 141 can be immersed in the culture medium.

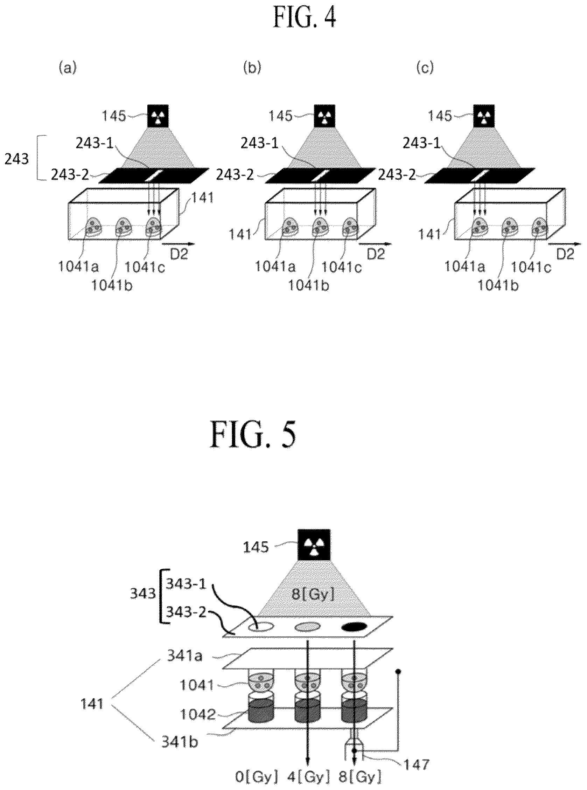

Hereinafter, various embodiments of the radiation dose control unit 143 will be described with reference to FIGS. 4 to 7.

Referring to FIG. 4, the radiation dose control unit 143 includes a slit type radiation dose modulator 243 having a radiation protection plate 243-2 having a slit 243-1. In this case, the radiation rays emitted from the radiation generating unit 145 is irradiated to the cancer organoid of the CRS plate 141 through the slit 243-1. The radiation protection plate 243-2 serves as a radiation shield, and the slit 243-1 is an open portion opened in a shape extending in the radiation protection plate 243-2. On the other hand, the relative position of the CRS plate 141 and the slit 243-1 may be moved perpendicularly to the longitudinal direction of the slit 243-1. However, in the embodiment of FIG. 4, the CRS plate 141 moves, but it is also possible to configure the slit 243-1 to move.

Meanwhile, the relative position of the slit 243-1 and the CRS plate 141 may be set in advance, and an overlap region of the slit 243-1 and the CRS plate 141 may be changed for a predetermined period of time.

More specifically, referring to FIG. 4, the CRS plate 141 may move in a second direction D2, namely, perpendicularly to the longitudinal direction of the slit 243-1, for the predetermined period of time. The predetermined period of time includes a first time interval, a second time interval, and a third time interval, and the CRS plate 141 is positioned as illustrated in FIG. 4A during the first time interval, such that a third organoid 1041C and the slit 243-1 overlap. Accordingly, radiation rays can be irradiated only to the third organoid 1041c. In addition, the CRS plate moves a the second direction D2 in the position of FIG. 4A, and is positioned as illustrated in FIG. 4B during the second time interval, such that the second organoid 1041b and the slit 243-1 overlap. Accordingly, radiation rays can be irradiated only to the second organoid 1041b. Further, the CRS plate 141 is moved in the second direction D2 from the position of FIG. 4B to be positioned as illustrated in FIG. 4C during the third time interval, such that the third organoid 1041c and the slit 243-1 overlap. Accordingly, radiation rays can be irradiated only to the third organoid 1041c.

In this instance, the radiation dose irradiated to each of the plurality of cancer organoids for the predetermined period of time may be proportional to the time in which each of the plurality of cancer organoid overlaps with the slit 243-1. That is, the total radiation dose irradiated to the first organoid 1041a is proportional to the first time interval, the total radiation dose irradiated to the second organoid 1041b is proportional to the second time interval, and the total radiation dose irradiated to the third organoid 1041c is proportional to the third time interval.

That is, the radiation sensitivity diagnosis kit 100 adjusts the overlapping period of time between each of the cancer organoids and the slit 243-1 by moving the CRS plate 141 in the second direction D2, thereby adjusting the total radiation doses irradiated to each of the cancer organoids for the predetermined period of time.

As illustrated in FIG. 4, in a case in which the CRS plate 141 is moved, when the radiation generating unit 145 and the slit 243-1 are fixed to make the radiation irradiation region and the radiation does constant and only the period of time is adjusted, the radiation dose can be adjusted accurately. However, it may be longer in radiation irradiation period of time than the radiation dose modulator 443.

Referring to FIG. 5, the radiation irradiating device 140 includes the radiation generating unit 145, a radiation dose control unit 143 including the density-adjustable radiation shield 343, and the CRS plate 141 including a first plate 341a and a second plate 341b.

In this embodiment, the density-adjustable radiation shield 343 includes a radiation-transmitting shielding plate 343-1 and a radiation protection plate 343-2 for receiving the radiation-transmitting shielding plate 343-1. In this case, the radiation dose applied to the cancer organoids 1041 can be selectively adjusted by changing the pattern, color, material (metal), density, etc. of the radiation-transmitting shielding plate 343-1 to control a radiation transmission rate. The radiation-transmitting shielding plate 343-1 is manufactured to fit an opening of a specific shape formed in the radiation protection plate 343-2. In this case, the radiation-transmitting shielding plate 343-1 of the density-adjustable radiation shield 343 is detachable from the radiation protection plate 343-2. The radiation protection plate 343-2 may use a film for completely shielding radiation rays, or use a film for transmitting radiation rays to serve only to physical support the radiation-transmitting shielding plate 343-1. The radiation protection plate 343-2 has a hole formed at a portion supporting the radiation-transmitting shielding plate 343-1 to selectively transmit radiation rays when using the film for completely shielding radiation rays.

Meanwhile, an amount of radiation rays transmitted through the density-adjustable radiation shield 343 may be varied according to types and radiation transmittance of the radiation-transmitting shielding plate 343-1. That is, depending on types of the radiation-transmitting shielding plate 343-1 attached to the radiation dose control unit, the radiation dose reaching the cancer organoid after transmitting the corresponding density-adjustable radiation shield 343 for the predetermined period time can be adjusted. Accordingly, the total radiation dose reaching at least a portion of the plurality of cancer organoids 1041 disposed on the CRS plate 141 for the predetermined period time can be adjusted differently according to the types (radiation transmittance) of the radiation-transmitting shielding plate 343-1 disposed to overlap with the cancer organoids. Accordingly, even though a single experiment using a plurality of cancer organoids 1041 is performed using one CRS plate 141, various radiation dose can be irradiated differently to the plurality of cancer organoids 1041, and thus, the viability of cancer cells can be measured under various radiation dose conditions.

For example, the CRS plate 141 is a unit which is arranged below the radiation dose control unit 143 and on which a female organoid (or cancer cell) is disposed, and includes a first plate 341a on which the cancer organoid is disposed, and a second plate 341b in which a culture medium 1042 for the growth of the cancer organoid is accommodated.

In this instance, the first plate 341a has a pillar structure, and the second plate 341b is in the form of a well having a larger diameter than the first plate 341a so as to receive the first plate 341a. However, the structure is not limited to the foregoing.

By this structural feature, even in the use of a single plate or the irradiation of a single radiation dose emitted from the radiation irradiating device 345, for example, 8Gy, it is possible to perform a cell experimental data-based analysis in accordance with experiments under conditions in which various radiation doses (eg, 0 Gy, 4 Gy, 8 Gy) are irradiated to the plurality of cancer organoids 1041.

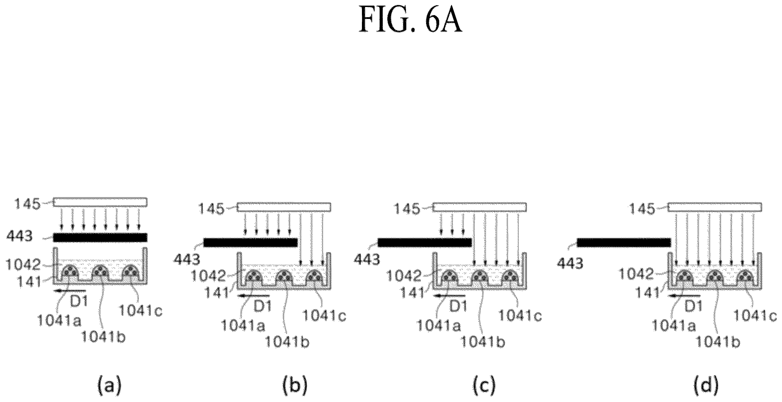

Referring to FIG. 6A, the radiation dose control unit 143 according to an embodiment of the present invention includes the shield movable type radiation dose modulator 443. Specifically, referring to FIG. 6A, the CRS plate 141 is disposed under a radiation protection plate 443-2 of the shield movable type radiation dose modulator 443. The radiation protection plate 443-2 is disposed between the CRS plate 141 and the radiation generating unit 145 in a state in which the CRS plate 141 is coupled to the remainder components of the shield movable type radiation dose modulator 443. That is, the radiation protection plate 443-2 of the shield movable type radiation dose modulator 443 is disposed between the CRS plate 141 and the radiation generating unit 145 and slides and moves along the flow of time to selectively shield the radiation rays emitted toward the CRS plate 141 from the radiation generating unit 145 according to time.

The radiation protection plate 443-2 of the shield movable type radiation dose modulator 443 slides in a first direction D1 which is an extension direction of the CRS plate 141, namely, in a direction perpendicular to a straight line connecting the radiation generating unit 145 and the CRS plate 141 or in a direction that one or more cancer organoids of the CRS plate 141 are arranged side by side. In other words, the radiation protection plate 443-2 of the shield movable type radiation dose modulator 443 slides in the direction parallel to the straight line connecting a first organoid, a second organoid, and a third organoid. The sliding action of the shield movable type radiation dose modulator 443 can be performed by a shield driving gear.

In a case in which radiation rays are irradiated from the radiation generating unit 145 to the CRS plate 141, the radiation rays are shielded in a region where the radiation protection plate 443-2 and the CRS plate 141 overlap, but are not shielded in a region where the radiation protection plate 443-2 and the CRS plate 141 do not overlap and are irradiated to the CRS plate 141. Therefore, as the radiation protection plate 443-2 moves along the first direction D1, it is controlled such that radiation rays are selectively irradiated to any one among the plurality of cancer organoids 1041 (first organoid to third organoid) disposed on the CRS plate 141. At least some of the radiation doses irradiated with respect to the one or more cancer organoids (the first organoid 1041a to the third organoid 1041c) may be different from one another.

For example, referring to FIGS. 6A to 6D, the radiation generating unit 145 continuously emits radiation rays toward the CRS plate 141 for a predetermined period of time. At this time, the radiation protection plate 443-2 of the shield movable type radiation dose modulator 443 slides gradually so as to be changed from the first state in which the radiation protection plate 443-2 of the shield movable type radiation dose modulator 443 is overlapped with all cancer organoids as illustrated in FIG 6A(a) to the second state in which the radiation protection plate 443-2 is not overlapped with all cancer organoids as illustrated in FIG 6A(d). In the first state, the first organoid 1041a, the second organoid 1041b, and the third organoid 1041c overlap with the radiation protection plate 443-2 so that radiation rays emitted from the radiation generating unit 145 may not reach. The radiation protection plate 443-2 in the first state may be moved in the first direction D1 as illustrated in FIG. 6A. Therefore, the first organoid 1041a and the second organoid 1041b overlap with the radiation protection plate 443-2 so that the radiation rays do not reach, but the third organoid 1041c is not overlapped with the radiation protection plate 443-2 so that radiation rays emitted from the radiation generating unit 145 can reach. Subsequently, the radiation protection plate 443-2 of FIG. 6A(b) is further moved in the first direction D1 as illustrated in FIG. 6A(c). Therefore, the first organoid 1041a is overlapped with the radiation protection plate 443-2 so that the radiation rays do not reach, but the second and third organoids 1041c so that the radiation rays emitted from the radiation generating unit 145 can reach. Subsequently, the radiation protection plate 443-2 of FIG. 6A(c) is further moved in the first direction D1 as illustrated in FIG. 6A(d) so as to be in the second state. Therefore, all of the first organoid 1041a, the second organoid 1041b, and the third organoid 1041c do not overlap with the radiation protection plate 443-2 so that the radiation rays can reach.

Through this process, the position and/or area where radiation rays are irradiated to the CRS plate 141 for a predetermined period of time can be changed. As the radiation protection plate 443-2 of the shield movable type radiation dose modulator 443 slides and moves in the first direction D1 for a predetermined period of time, the area in which radiation rays are irradiated to the CRS plate 141 can be increased. Accordingly, the total radiation dose irradiated for the predetermined period of time to the first organoid 1041a of the CRS plate 141 is smaller than the total radiation dose irradiated for the predetermined period of time to the second organoid 1041b, and the total radiation dose irradiated for the predetermined period of time to the second organoid 1041b may be smaller than the total radiation dose irradiated for the predetermined period of time to the third organoid 1041c.

As described above, as time goes by, the radiation protection plate 443-2 of the shield movable type radiation dose modulator 443 slides in the first direction D1 so that the relative position between the CRS plate 141 and the radiation protection plate 443-2 is changed, and the total radiation dose irradiated to a portion of the plurality of organoids arranged on the CRS plate 141 for the predetermined period of time may be adjusted to be different from each other. Therefore, the total radiation dose irradiated to the plurality of cancer organoids 1041 in a biological experiment of the condition in which the radiation rays of the same intensity is emitted during the same predetermined period of time may be different. Therefore, the present invention provides an effect capable of obtaining data based on different total radiation doses and lots of cell experiments even tough experiments are performed during a limited period of time (predetermined period of time) using a small number of radiation irradiation devices 140.

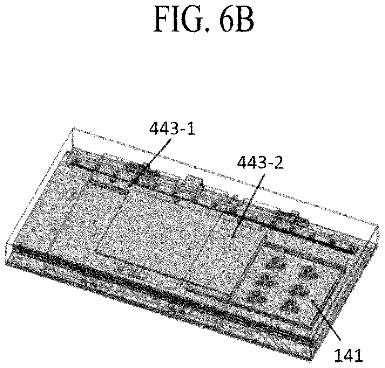

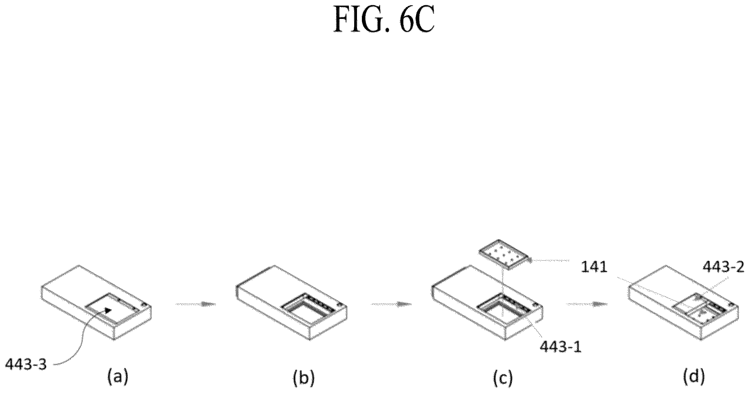

Referring to FIGS. 6B and 6C, the shield movable type radiation dose modulator 443 will be described in more detail. The shield movable type radiation dose modulator 443 includes a shield driving gear 443-1 and a radiation protection plate 443-2. The shield movable type radiation dose modulator 443 can detach the CRS plate 141 as illustrated in FIG. 6C. The shield driving gear 443-1 is a linear actuator for linearly moving the radiation protection plate 443-2, and the radiation protection plate 443-2 performs a linear motion to expose a specific area of the CRS plate 141 or to shield radiation rays for the predetermined period of time with respect to the specific area of the CRS plate 141.

Referring to FIG. 6C, in order to detach the CRS plate 141, the shield movable type radiation dose modulator 443 may first remove a cover 443-3 (see FIGS. 6C(a) and 6C(b)), and then, mount the CRS plate 141 in a side space of the shield driving gear 443-1 (see FIGS. 6C(c) and 6C(d)).

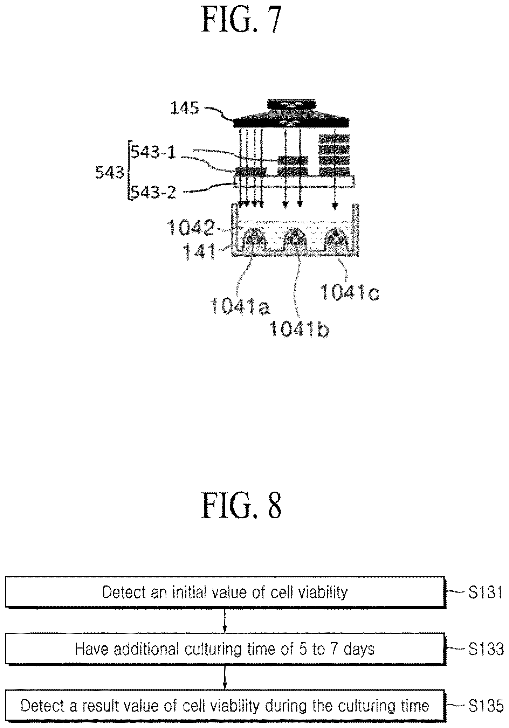

Referring to FIG. 7, the radiation dose control unit 143 includes a laminated radiation shield 543. The laminated radiation shield 543 includes a radiation transmission shielding plate 543-1 and a radiation protection plate 543-2 for receiving the radiation transmission shielding plate 543-1, and is configured to selectively shield the radiation rays emitted from the radiation generating unit 145, that is, is configured to transmit some of the radiation rays. The radiation protection plate 543-2 uses a film for completely shielding radiation rays, and just serves to physically support the radiation transmission shielding plate 543-1 using the film transmitting the radiation rays. When the radiation protection plate 543-2 is used to completely shield the radiation rays, the radiation protection plate 543-2 has a hole formed at a portion supporting the radiation transmission shielding plate 543-1 to selectively transmit radiation rays.

The radiation transmission shielding plate 543-1 of the laminated radiation shield 543 may be made of a material including, for example, tungsten, to selectively shield radiation rays.

The plurality of cancer organoids 1041 of the CRS plate 141 are different in the number of the radiation transmission shielding plates 543-1 stacked, and the size of the radiation dose irradiated corresponding to the number of the radiation transmission shielding plates 543-1 stacked is determined.

For example, as illustrated in FIG. 7, a sheet of the radiation transmission shielding plates 543-1 is stacked on the first organoid 1041a, two sheets of the radiation transmission shielding plates 543-1 are stacked on the second organoid 1041b, and three sheets of the radiation transmission shielding plates 543-1 are stacked on the second organoid 1041c. When the number of the radiation transmission shielding plates 543-1 stacked is increased, the intensity of the radiation rays irradiated to the corresponding organoid is reduced.

In this instance, as the number of the radiation transmission shielding plates 543-1, for instance, tungsten sheets, of a unit thickness, for instance, 1 mm, increases, the radiation dose (in the unit of Gy) per minute irradiated to the cancer organoid 1041 can be reduced, and the total radiation dose irradiated for a predetermined period of time is reduced as the number of the radiation transmission shielding plates 543-1, for instance, tungsten sheets, having a thickness of 1 mm increases.

The thickness of the radiation transmission shielding plates 543-1 may be changed by a user's selection, if necessary. As the thickness of the radiation transmission shielding plates 543-1 is smaller, a difference in total radiation dose irradiated to the cancer organoids 1041 can be adjusted more precisely. In this instance, the thicknesses of all the radiation transmission shielding plates 543-1 are constant, for example, 1 mm. However, the present invention is not limited thereto, and the thicknesses of all the radiation transmission shielding plates 543-1 may not be constant.

When the number of the radiation transmission shielding plates 543-1 stacked on the plurality of cancer organoids 1041 in the overlapping region is adjusted, the total radiation dose irradiated to the plurality of cancer organoids 1041 for the predetermined period of time can be adjusted.

That is, the radiation generating unit 145 irradiates a predetermined intensity of radiation rays toward the CRS plate 141 for a predetermined period of time, and changes the number of the radiation transmission shielding plates 543-1, if necessary, so that the total radiation dose irradiated to each of the plurality of cancer organoids 1041 (the total radiation dose irradiated for a predetermined period of time) can be adjusted.

The control unit 150 includes a storage unit 110, a processor 120, a communication unit 130, and a radiation sensitivity diagnosis module 135, which is a radiation diagnosis software module. The control unit 130 is software installed in the control unit 150, which is a computer, but is not limited thereto. It will be appreciated by those skilled in the art that the radiation sensitivity diagnosis module 135 may be implemented in a variety of ways, such as a software module, a hardware module, a combination module of software and hardware.

In this case, the storage unit 110 may store various data generated during the diagnosis of the degree of sensitivity of radiation rays to an object. For example, the storage unit 110 is configured to store the cell experiment-based data extracted by the radiation diagnosis module 135, the radiation response factor, the cell growth factor, and the diagnosis result of the sensitivity level. The stored cell experiment-based data may be applied as reference data. In various embodiments, the storage unit 110 may be at least one among a flash memory type, a hard disk type, a multimedia card micro type, a card type memory (e.g., an SD wallet or an XD memory, etc.), a RAM, an SRAM, a ROM, an EEPROM, a PROM, a magnetic memory, a magnetic disk, and an optical disk.

The communication unit 130 connects the radiation sensitivity diagnosis kit 100 and an external system to be able to communicate. The communication unit 130 can transmit and receive various data by being connected to a user terminal 200 and a data base providing server 300 using wired/wireless communication. Specifically, the communication unit 130 receives data from the radiation irradiating device 140, or receives reference data, which is a clinical outcome or a cell experiment-based data of another individual from the database providing server 300. Further, the communication unit 130 transmits a diagnosis result to the user terminal 200.

The processor 120 is operatively connected to the storage unit 110, the communication unit 130, and the radiation irradiating device 140, executes a radiation sensitivity diagnosis module 135 to obtain cell experiment-based data for cancer cells, analyzes the experiment-based data to extract an associated sensitivity feature, and performs various instructions to determine the degree of radiation sensitivity based on the analysis.

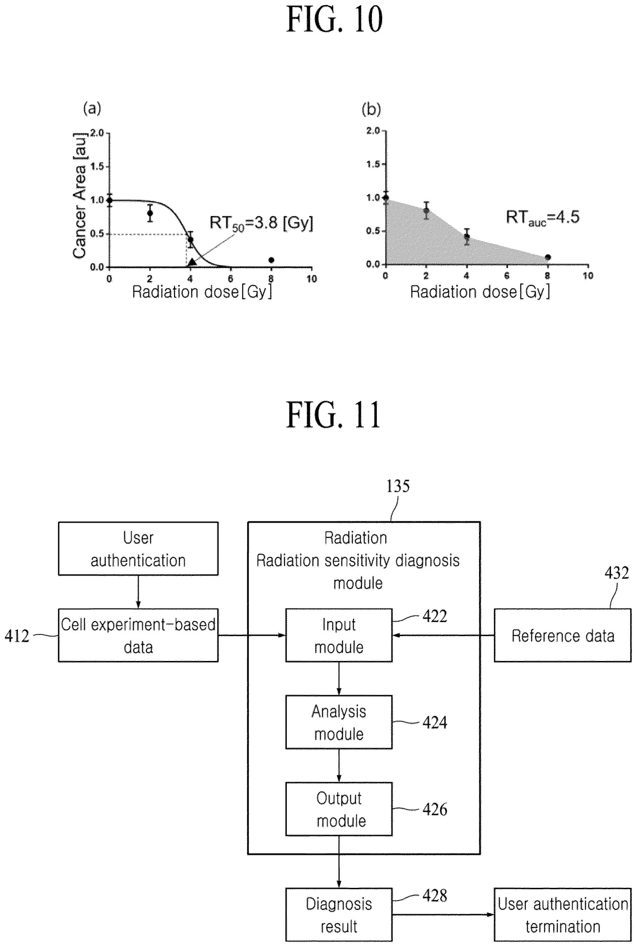

The radiation diagnosis module 135 is executed by the processor, controls the radiation irradiating device 140, the radiation generating unit 145, and the radiation control unit 143, and controls to store the cell experiment-based data to the storage unit. Referring to FIG. 11, a specific implementation of the radiation diagnosis module 135 will be described later.

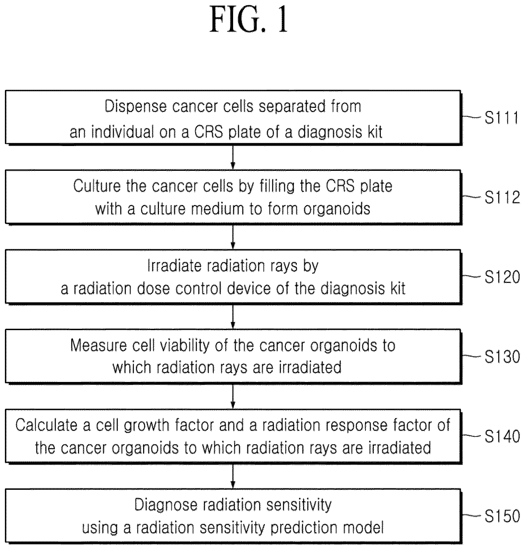

Hereinafter, a radiation sensitivity diagnosis method according to an embodiment of the present invention will be described in detail with reference to FIGS. 1, 3A and 3B.

The radiation sensitivity diagnosis method according to an embodiment of the present invention includes the steps of: (a) dispensing one or more cancer cells separated from an individual on a CRS plate of the diagnosis kit (S111); (b) culturing the at least one cancer cell to form one or more cancer organoids by filling the CRS plate with a culture medium or immersing the CRS plate in a well (S112); (c) irradiating radiation rays, which passed a radiation dose control unit, to the CRS plate from the radiation generating unit of the radiation irradiating device; (d) measuring cell viability of the at least one cancer organoid (S120); (e) calculating a radiation response factor and a cell growth factor of the at least one cancer organoid on the basis of the measured cell viability (S130); and (f) diagnosing radiation sensitivity for the individual by the control unit based on the radiation response factor and the cell growth factor of the at least one cancer organoid.

Specifically, referring to FIG. 1, the radiation sensitivity diagnosis method according to an embodiment of the present invention first includes the step of (a) dispensing one or more cancer cells separated from an individual, for instance, a patient, on a CRS plate of the diagnosis kit (S111).

In this case, the cancer cells are prepared as follows. First, cancer tissue is extracted from a patient, and the extracted cancer tissue is enzyme-treated to make a single cancer cell. Blood cells (red blood cells/leukocytes/platelet and the like) are removed from the enzyme-treated single cancer cells, and the single cancer cells from which the blood cells are removed are counted and mixed with the extracellular matrix. In addition, the single cancer cells are dispensed on the CRS plate 141. It will be understood by those skilled in the art that the scope of the present invention is not limited thereto and that the method of dispensing cancer cells may be performed by various methods.

Specifically, referring to FIGS. 3A and 3B, the cancer cells are dispensed at ends of a plurality of pillar portions 141-1. In this instance, the cancer cells can be dispensed by, for example, a 3D bio printer, for instance, ASFA™ Spotter.

Then, the CRS plate 141 or a well 141-3 is filled with a culture medium to culture the cancer cells (S112). The CRS plate 141 may include a well (see FIG. 3A) or may require a separate well (see FIG. 3B).

Specifically, in step (b) of FIG. 3A, culture medium is put in the CRS plate 141 having a cell-divided pillar. On the other hand, in step (b) of FIG. 3B, the CRS plate 141 is immersed in the well 141-3 having the culture medium. In this instance, the cancer organoid 1041 is generated while the cancer cells are cultured. In step (b) of FIGS. 3A and 3B, the cancer cells dispensed on the CRS plate 141 may be, for example, a first cancer organoid, a second cancer organoid, and a third cancer organoid in order from the left.

Next, radiation rays are irradiated to the cancer organoid 1041 by the radiation irradiating device 140 of the radiation sensitivity diagnosis kit 100 (S120), and then, a step of taking out the CRS plate 141 on which the radiation rays are irradiated is performed.

However, it is not necessary to take out the CRS plate 141, and it is also possible to obtain an image of the cancer organoid 1041 without taking out the CRS plate 141 according to the structure of the radiation irradiating device 140. For example, in the case of FIG. 3B in which the CRS plate 141 not including a well is immersed in a separate well, the CRS plate 141 is taken out to obtain an image by the imaging device 147, and the image is analyzed to calculate cell viability.

Specifically, referring to the step (C) of FIGS. 3A and 3B, the radiation generating unit 145 controls to diversify the radiation doses irradiated to the at least one cancer organoid by the radiation dose control unit 143, for instance, to diversify the radiation doses applied to a portion of the at least one cancer organoid, so as to irradiate radiation rays. In this case, the radiation dose control unit 143 may have various types. For instance, the radiation dose control unit 143 includes a slit type radiation dose modulator 243 for controlling the radiation doses through a slit 243-1 relatively moving with respect to the CRS plate 141, a shield movable type radiation dose modulator 443 for controlling radiation irradiation time for each cancer organoid by moving a shield in a rectilinear motion, a density-adjustable radiation shield 343 configured to selectively adjust the radiation doses according to the radiation transmittance controlled by the pattern, color, material (metal), material density, and the like of radiation transmitting shield plates, and a laminated radiation shield 543 varying the number of the radiation transmitting shield plates according to the radiation doses to be adjusted.

Radiation rays with different radiation doses may be irradiated to the plurality of cancer organoids 1041, for instance, the first cancer organoid, the second cancer organoid, and the third cancer organoid, through the radiation dose control unit 143. In addition, radiation rays may not be irradiated to any one of the plural cancer organoids 1041.

The radiation sensitivity diagnosis kit 100 measures cell viability for the cancer organoid to which radiation rays are irradiated (S130).

Cell viability is measured, for example, by measuring the total strength/size or selected strength/size of a fluorescent material by dyeing living cells, or by counting dead cells by dyeing dead cells. Alternatively, cell viability may be measured by measuring the transduction of cells or a solution containing cells using chemical elements such as MTT or APT.

In this instance, the cell viability is required in various cases. First, in order to measure cell viability, before radiation rays with the plurality of radiation doses are irradiated, an initial value of the cell viability of the cancer organoid 1041 is measured. After additional culturing time of five days to seven days, a result value of the cell viability during the culturing time is measured. Therefore, a radiation response of the cancer organoid 1041 can be confirmed by obtaining the cell viability according to the time and the irradiated radiation dose.

**Mode for Invention**

Referring to FIG. 8, in order to measure cell viability, the initial value of the cell viability of the cancer organoid 1041 irradiated with radiation rays is measured. After measuring (S131), it is preferable to have culturing time of five to seven days. Finally, the result value of the cell viability during the culturing time is measured.

Therefore, cell viability according to time and irradiated radiation dose can be obtained.

Thereafter, a cell growth factor and a radiation response factor of the cancer organoid irradiated with radiation rays are calculated based on the measured cell viability. More specifically, the radiation sensitivity diagnosis kit 100 can obtain cell experiment-based data by calculating the cell growth factor and the radiation response factor based on the measured cell viability.

The cell experiment-based data may refer to test result data, test condition data, etc., based on a cell experiment performed by the radiation irradiating device 140 using cancer cells (cancer organoid) separated from an individual. For example, the cell experiment-based data may be raw data associated with cell viability in accordance with kinds of radiation rays, intensity of radiation, cell viability, and cancer cell radiation therapy of the radiation response factor and the cell growth factor, or may a radiation processing condition data. However, the cell experiment-based data is not limited to those described above. In addition, the cell experiment-based data should be understood to encompass all data obtained through clinical trials as well as data obtained through treatment or diagnosis.

The cell growth factor includes a quantitative indicator indicating growth of cancer cells according to time. The cell growth factor can encompass both a factor and a statistical index (Z score) of the factor capable of indicating cell proliferation, such as a cell growth rate. For instance, the cell growth factor can be obtained by measuring cell viability according to time. The cell growth factor can be a cell viability increase rate, a cell growth rate, and a cell size increase rate. At this time, the cell growth rate may mean a change in volume, size, and the number of cells for a predetermined period of time.

The radiation response factor may include a cell viability according to a change in radiation dose as a criterion for quantitatively measuring the reaction of cells to radiation rays. The radiation response factor can encompass both a factor and a statistical index (Z score) of the factor capable of indicating radiation effects, such as $RT_{50}$, $\%RT_{50}$, $RT_{AUC}$ (area of a radiation response curve) cell viability, cell viability change rate, and the like. For example, $RT_{50}$ is a radiation response factor corresponding to the radiation dose which is reduced to 50% in the cell viability of cancer cells when radiation rays are not irradiated, and the $RT_{AUC}$ is a radiation response factor corresponding to the area of a cell viability curve according to the radiation dose, namely, an integral area, i.e., the area surrounded by the X-axis, the Y-axis, and a cell viability curve.

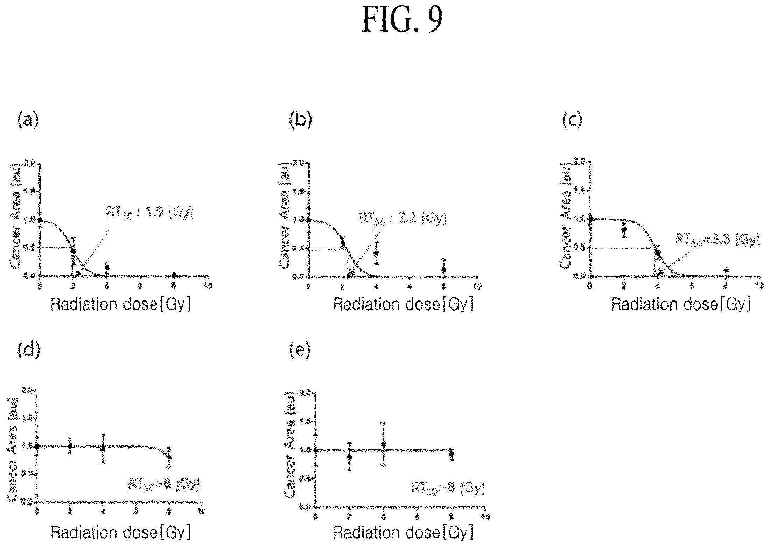

More specifically, referring to FIGS. 9 and 10, the radiation response factor of the $RT_{50}$ value and the $RT_{AUC}$ value may be provided as a radiation response factor.

$RT_{50}$ and $\%RT_{50}$ may mean radiation response factors corresponding to the radiation dose reduced to 50% in viability of cancer cells. FIGS. 9A to 9E, and FIG. 10A illustrate examples of $RT_{50}$ radiation response factors in various cases. In this embodiment, the $RT_{50}$ was calculated based on the cell viability (cancer cell area) according to radiation doses. In FIG. 9A, the $RT_{50}$ value, which is a radiation dose reduced to 50% in viability of cancer cells, is 1.9 Gy, and FIG. 9B shows the $RT_{50}$ value of 2.2 Gy, and FIG. 9C shows the $RT_{50}$ value of 3.8 Gy.

Meanwhile, in FIGS. 9D and 9E, the $RT_{50}$ value is higher than 8 Gy. Since the radiation generating unit 145 according to an embodiment of the present invention can generate a radiation dose ranging 0 Gy to 8 Gy, the $RT_{50}$ value cannot be measured, and in this instance, radiation sensitivity is lower than those of FIGS. 9A, 9B and 9C.

As another example, the $RT_{AUC}$ is a radiation response factor corresponding to the area of a cell viability curve according to the radiation dose, namely, an integral area, i.e., the area surrounded by the X-axis, the Y-axis, and a cell viability curve. FIG. 7B shows that the $RT_{AUC}$ which is an integral area of a portion surrounded by the cell viability curve is 4.5.

Referring back to FIG. 1, the radiation sensitivity diagnosis method according to an embodiment of the present invention generates a radiation sensitivity diagnosis model based on the calculated cell growth factor and the radiation response factor, and diagnoses radiation sensitivity using the generated sensitivity prediction model (S150).

In this case, the radiation sensitivity diagnosis module 135 of FIG. 11 can generate the radiation sensitivity diagnosis model and diagnose radiation sensitivity of an individual patient or an individual sample based on the generated radiation sensitivity diagnosis model.

The radiation sensitivity diagnosis module 135 is, for example, a software module, stored in the storage unit 110 and executed by the processor 120. Meanwhile, the radiation sensitivity diagnosis module 135 can extract a sensitivity feature using the cell experiment-based data to generate a radiation sensitivity diagnosis model. The radiation sensitivity diagnosis module 135 can also determine the radiation sensitivity (or radiation therapy response prediction) for an individual (such as an individual patient or an experimental sample) as positive or negative by the radiation sensitivity diagnosis model.

Specifically, referring to FIG. 11, the radiation sensitivity diagnosis module 135 may be learned to receive a cell experiment-based data, which is associated with a radiation response factor and a cell growth factor of, for example, an individual and/or another individual, as reference data 432, and extract a sensitivity feature from the data to generate a radiation sensitivity diagnosis model.

The radiation sensitivity diagnosis module 135 is configured to receive reference data 432, which is cell experiment-based data, which is a clinical result and/or a measurement result of another patient or individual, and the cell experiment-based data of a specific entity, and diagnose radiation sensitivity of a specific individual based on the sensitivity feature and the reference data.

When the radiation sensitivity diagnosis module 135 diagnoses the radiation sensitivity of a specific individual for radiation therapy, after a user who wants to know sensitivity of the specific individual is authorized through log-in, the cell experiment-based data 412 of the received specific individual is input to the susceptibility diagnostic module 135. At this time, the cell experiment-based data 412 of the specific individual may be input by an input module 422 of the radiation sensitivity diagnosis module 135. The input module 422 may further receive the reference data 432.

After that, the sensitivity feature is extracted from the cell experiment-based data 412 of the specific individual by the analysis module 424, and the degree of radiation sensitivity may be determined and output based on the sensitivity feature by the output module 426. In this instance, the output module 426 outputs a radiation sensitivity diagnosis result for the specific individual together with the sensitivity feature extracted from the cell experiment-based data 412 of the specific individual using the reference data 432.

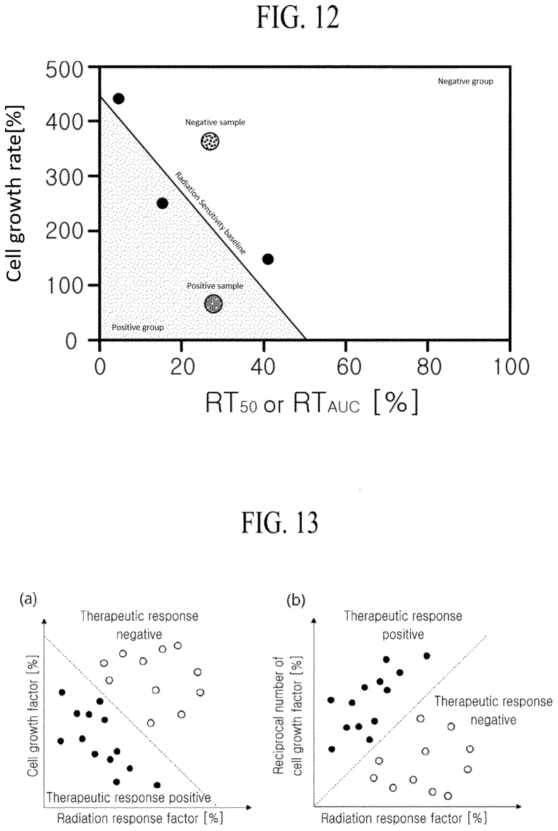

For example, the analysis module 424 and the output module 426 output the radiation sensitivity diagnostic result as a therapeutic response positive or a therapeutic response negative based on multiple factors of the radiation response factor and the cell growth factor. It is described below with reference to FIGS. 12 to 14.

Alternatively, the analysis module 424 and the output module 426 may determine the radiation sensitivity (degree of radiation therapy) for the individual as good, fair, and poor, and provide an analysis result 428. For example, the analysis module 424 is configured to calculate the radiation sensitivity index from the cell experiment-based data 412 of a specific individual. The output module 426 determines the treatment response degree as 'poor' in a case in which the radiation sensitivity index ranges from -100% to -10%, determines the treatment response degree as 'fair' in a case in which the radiation sensitivity index ranges from -10% to +10%, and determines the treatment response degree as 'good' in a case in which the radiation sensitivity index ranges from +10% to +100% according to a predetermined standard.

Alternatively, the analysis module 424 and the output module 426 may stochastically output the therapeutic reactivity. Furthermore, the output module 426 may further determine an efficacy diagnostic diagram of the radiation for indicating the degree of sensitivity to radiation in a table.

The radiation sensitivity diagnosis module 135 provides a diagnostic result of radiation sensitivity for the individual to the user terminal and terminates the user's authentication.

Specifically, the radiation sensitivity diagnosis module 135 may provide the degree of sensitivity (good, fair, poor) to radiation of an individual, whether the therapeutic response is positive, and an effect diagnosis table of radiation rays showing the degree of sensitivity to radiation to the user.

For example, the diagnosis result 428 of FIG. 11 is transmitted to the user terminal by the output module 426 and provided through a display unit of the user terminal.

On the other hand, according to another aspect of the present invention, user authentication termination (log-out) is performed after information on radiation sensitivity is provided.

Hereinafter, the radiation sensitivity diagnosis model of the analysis module 424 will be described in detail with reference to FIGS. 12 through 14. FIGS. 12 through 14 are diagrams illustrating a radiation sensitivity diagnosis model according to an embodiment of the present invention.

It has been noted that the inventors of the present invention have performed a wide range of studies on which one, among multiple factors of cancer cells, such as the cell growth factor and the radiation response factor, is related to the radiation therapy sensitivity of a patient. They noticed that prediction of the radiation therapy effect is the most accurate when considering the cell growth factor and the radiation response factor at the same time.

Specifically, according to an embodiment of the present invention, as illustrated in FIG. 12, a graph in which the radiation response factor and the cell growth factor are respectively set as an x-axis and a y-axis is provided as a radiation sensitivity diagnosis model.

The radiation sensitivity diagnosis model determines radiation sensitivity based on the sensitivity feature including a sensitivity baseline according to radiation rays. In the present model, the radiation sensitivity is not determined just by the radiation response factor, but is determined on the basis of the radiation sensitivity baseline formed of the radiation response factor and the cell growth factor. As illustrated in FIG. 12, the radiation sensitivity may be determined differently, namely, determined as a positive (with treatment effect) and a negative (without the treatment effect), depending on the radiation sensitivity baseline in which the cell growth factor (cell growth rate) is considered, even though a sample has the same radiation response factor ($RT_{50}$ or $RT_{AUC}$).

As described above, the radiation sensitivity baseline (fitting line) for determining the radiation reactivity into a positive group and a negative group may be formed, for example, to reduce the cell growth factor (%) as the radiation response factor ($RT_{50}$ value or $RT_{AUC}$ value) increases. Thus, the cell growth factor (%) dividing the radiation reactivity into a positive group and a negative group can be reduced when the radiation response factor ($RT_{50}$ value or $RT_{AUC}$ value) is increased. Accordingly, even though having the same radiation response factor ($RT_{50}$ value), the individual can be classified into a positive (treatment effect) and a negative (no therapeutic effect) for radiation therapy according to a cell growth factor of the corresponding individual (sample).

In this case, for example, the radiation sensitivity baseline of the sensitivity feature may be a fitting line created by performing curve fitting with respect to the cell experiment-based data including the cell growth factor and the radiation response factor. According to an embodiment of the present invention, the curve fitting was performed using a multiple factor regression analysis method.

The curve fitting is not limited thereto. In step (S150) of diagnosing the sensitivity to radiation, the curve fitting is carried out by at least one among, LR (LogistRT regression), PR (Probit regression), QuadratRT classifiers, Kernel estimation, LVQ(Learning vector quantization), ANN(ArtifRTial neural networks), RF(random forest), Bagging(bootstrap aggregating), AdaBoost, Gradient Boosting, XGBoost, SVM(support vector machine), LASSO(least absolute shrinkage and selection operator), Ridge(ridge regression), and ElastRT Net. In this instance, the degree of radiation sensitivity may be determined by the fitting line cut by the curve fitting.

Referring to FIG. 13, the radiation sensitivity diagnosis model is selected among a model (FIG. 13(a)) for diagnosing a therapeutic reaction on the basis of the radiation response factor and the cell growth factor, and a model (FIG. 13(b)) for diagnosing a therapeutic reaction on the basis of a reciprocal number of the cell growth factor and the radiation response factor. That is, as illustrated in FIG. 12, a model for diagnosing the therapeutic reaction (positive group or negative group) on the basis of the graph of FIG. 13(a) in which the radiation sensitivity baseline is inverse proportional according to the relationship between the radiation response factor (%) and the cell growth factor (%) may be used. Alternatively, a model for diagnosing the therapeutic reaction (positive group or negative group) on the basis of the graph of FIG. 13(b) in which the radiation sensitivity baseline (fitting line) is proportional according to the relationship between the reciprocal number (1/%) of the cell growth factor and the radiation response factor.

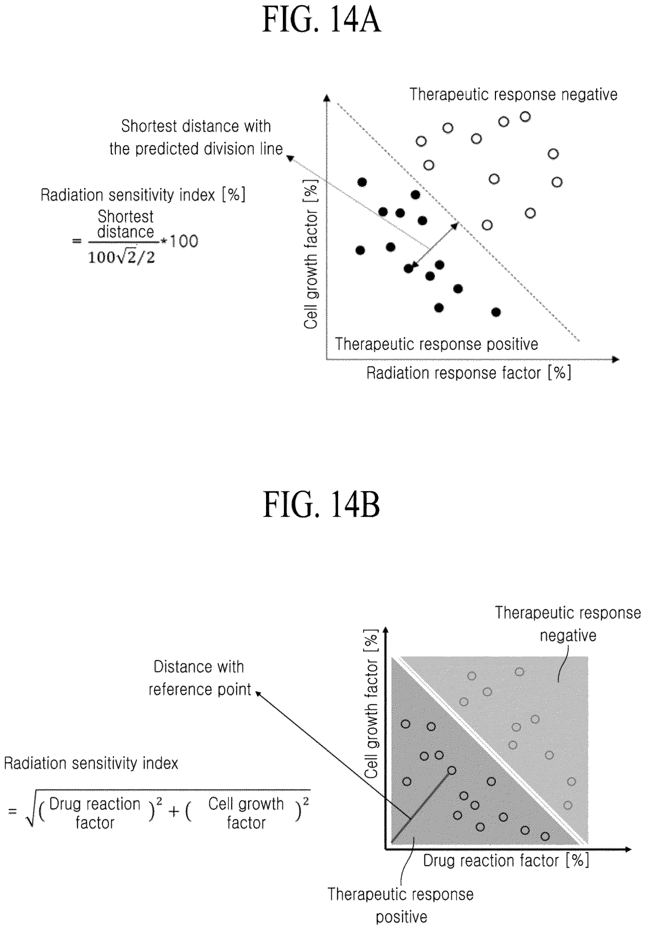

Referring to FIG. 14A, in the model for diagnosing the therapeutic reaction on the basis of the radiation response factor and the cell growth factor (FIG. 12 and FIG. 13(a)), the radiation sensitivity index can be calculated by the following Equation, based on the determined radiation sensitivity baseline, or the shortest distance between the fitting line and new data:

[Equation 1]

$$\text{Radiation Sensitivity Index (\%)} = \frac{\frac{\text{Shortest distance}}{100\sqrt{2}/2}}{} * 100[\%].$$

That is, the radiation sensitivity index can be determined according to the shortest distance between the position of the individual by the cell growth factor and the radiation response factor and the predicted division line (fitting line).

In this instance, in order to normalize the radiation sensitivity index with a percentage, the maximum distance is a half of $100\sqrt{2}$, which is a distance between 100% of the X-axis and 100% of the Y-axis. Accordingly, the ratio [%] to $100\sqrt{2}/2$ of the shortest distance from the predicted division line can be determined as the radiation sensitivity index.

The shortest distance from the predicted division line may be a positive number in an area (adjacent to an intersection of the X-axis and the Y-axis) in which individuals having a therapeutic response positive based on the predicted division line are arranged, and may be a negative number in an area (spaced apart from the intersection of the X-axis and the Y-axis) in which individuals having a therapeutic response negative based on the predicted division line are arranged. Therefore, the radiation sensitivity index may be zero in the predicted division line, may be positive in the therapeutic response positive region based on the prediction division line, and may be negative in the therapeutic response negative region based on the predicted division line. In addition, as the shortest distance from the prediction division line increases, that is, as the individual is far from the prediction division line, an absolute value of the radiation sensitivity index can be increased. Thus, the radiation sensitivity index may have a value ranging from -100% to +100%.

As another example, in the normalization, the half distance between the X-intercept and the Y-intercept of the radiation sensitivity baseline can be used as the maximum distance, or a half ( $100\sqrt{2}$ in FIG. 13A) of the distance connecting the maximum values of the X-axis and the Y-axis in the data range can be used.

Referring to FIG. 14B which is another embodiment of the present invention, in the model for diagnosing a therapeutic reaction on the basis of the radiation response factor (drug reaction factor) and the cell growth factor (FIG. 12 and FIG. 13(a)), the radiation sensitivity index can be calculated by the following Equation 2 based on a distance (distance to a reference point) with a reference point (intersection between the cell growth factor axis and the drug reaction factor axis). In this instance, the reference point may be the origin (0, 0), and in some cases, other values can be added.

[Equation 2]

$$\text{Radiation Sensitivity Index (\%)} = \sqrt{(\text{Drug reaction factor})^2 + (\text{Cell growth factor})^2}$$

That is, the radiation sensitivity index can be determined according to the shortest distance between the position of the individual and the reference point in accordance with the cell growth factor and the radiation response factor.

Meanwhile, the radiation sensitivity diagnosis module 135 is configured to determine what region it belongs to, among the therapeutic response positive region, the therapeutic response negative region, and an uncertainty region according to the radiation sensitivity index obtained as described above. The uncertainty region may be a region corresponding to an individual that does not belong to any one among the positive region and the negative region. The uncertainty region is an area where the shortest distance from the predicted division line is less than a predetermined distance. That is, if the shortest distance from the predicted division line is less than a predetermined distance regardless of what region it belongs to, on the basis of the predicted division line, it is possible to determine it as an uncertain individual without determining a positive or a negative. Accordingly, the corresponding individual can be excluded from diagnosis because it is not determined. Thus, the present invention can increase accuracy in the overall therapeutic response prediction.

For example, according to an embodiment, the radiation sensitivity diagnosis module 135 may determine determines the treatment response degree as 'poor' in a case in which the radiation sensitivity index ranges from -100% to -10%, determines the treatment response degree as 'fair' in a case in which the radiation sensitivity index ranges from -10% to +10%, and determines the treatment response degree as 'good' in a case in which the radiation sensitivity index ranges from +10% to +100%. That is, the treatment response degree is determined as good/fair/poor according to the predetermined standard to perform sensitivity diagnosis.

The radiation sensitivity diagnosis kit 100 according to various embodiments of the present invention can predict radiation sensitivity with high accuracy. Accordingly, the radiation sensitivity diagnosis kit 100 according to the present invention allows a medical staff to quickly select an appropriate treatment radiation dose according to a diagnosis result, and thus, the radiation sensitivity diagnosis system of the present invention can contribute to early treatment and good treatment prognosis.

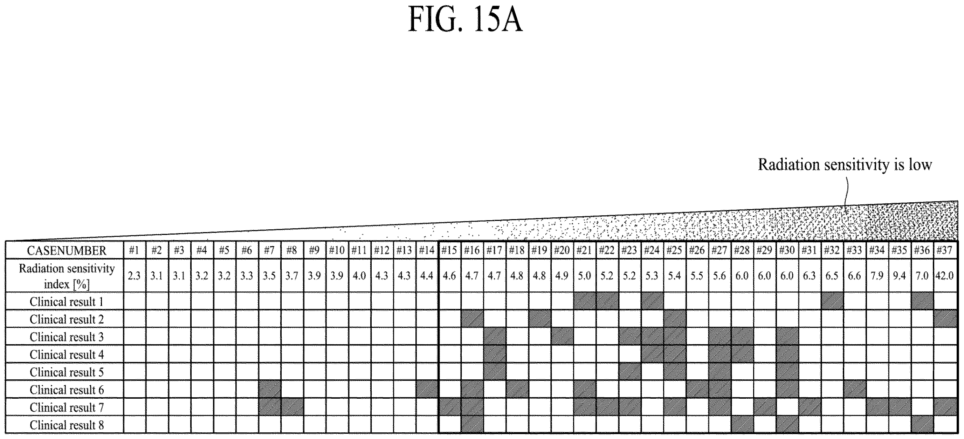

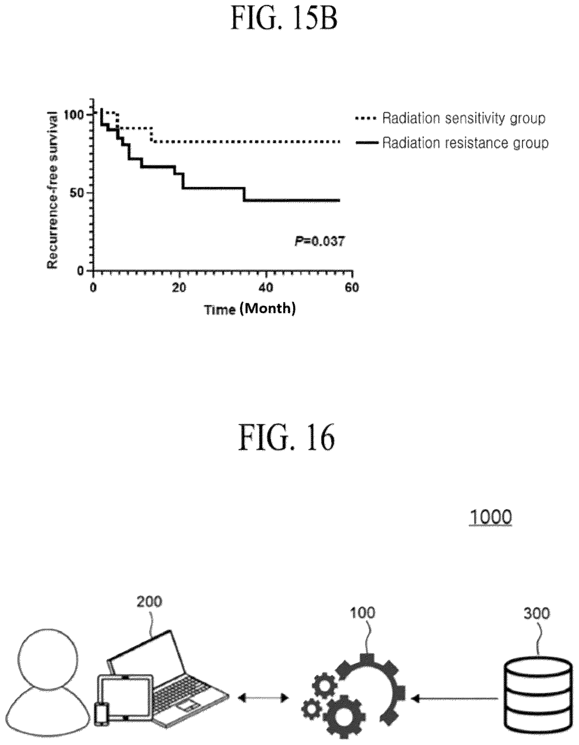

Hereinafter, the result that radiation sensitivity for various individuals is diagnosed using the radiation sensitivity diagnosis kit 100 described above will be described with reference to FIGS. 15A and 15B.

Referring to FIG. 15A, the table of FIG. 15A shows radiation sensitivity diagnosis results and clinical parameters of a head-neck cancer patient. The longitudinal axis of the table indicates a clinical result (patients #15 to #37 included in the item indicated by a bold line were not treated well due to low radiation sensitivity), and the horizontal axis of the table represents the sensitivity index of head and neck cancer patients. Clinical Results 1 to 8 mean clinical results capable of determining the prognosis of patients, such as, whether to recur after surgery, whether to be undivided, or the like. Through the clinical results illustrated in FIG. 15A, it has been found out that a higher value of the radiation response factor showed a bad clinical result of the cancer patient, and thus, it showed low radiation sensitivity.

FIG. 15B shows a survival rate of head and neck cancer patients according to a patient group sensitive to radiation rays (radiation sensitivity group, treatment response positive group), and a patient group unsensitive to radiation rays (radiation resistance group, treatment response negative group) according to radiation index. The patient group sensitive to radiation rays showed a high survival rate which is statistically meaningful when being compared in the survival rate for 60 months.

These results propose that the sensitivity index based on the radiation response factor and/or the cell growth factor can be an index to diagnose radiation sensitivity.

Continuously, a radiation sensitivity diagnosis system 1000 according to an embodiment of the present invention, including a radiation sensitivity diagnosis kit 100, will be described in detail.

FIG. 16 illustrates a radiation sensitivity diagnosis system 1000 according to an embodiment of the present invention. Specifically, referring to FIG. 16, the radiation sensitivity diagnosis system 1000 is a system that generates cell experiment-based data for cancer cells, determines radiation sensitivity to an individual of cancer cells using such data, and provides related information.

The radiation sensitivity diagnosis system 1000 includes a radiation sensitivity diagnosis kit 100 configured to determine the degree of radiation sensitivity to an individual based on cell experiment-based data, a user terminal 200 that receives information on the radiation sensitivity, and a database providing server 300 that provides cell experiment-based data and/or clinical outcome reference data.

In this case, the radiation sensitivity diagnosis kit 100 includes a radiation irradiating device 140 and a control unit 150, wherein the control unit 150 includes a general purpose computer, a laptop, and/or a data server that perform various operations to diagnose the degree of radiation sensitivity based on the cell experiment-based data and/or reference data of a specific user provided from the radiation irradiating device 140 and/or the data base providing server 300.

The radiation sensitivity diagnosis kit 100 provides diagnosis information on the radiation sensitivity for the individual to the user terminal 200. The data provided from the radiation sensitivity diagnosis kit 100 is provided to a web page through a web browser installed in the user terminal 200, or is provided in the form of an application or a program. In various embodiments, such data is provided in a form contained in a platform in a client-server environment.

On the other hand, the user terminal 200 is an electronic system for requesting provision of information on radiation sensitivity for an individual and providing a user interface to show diagnosis result data, and includes at least one among a smartphone, a tablet PC, a notebook, a personal computer (PC), etc. The user terminal 200 is used by a user, such as a medical team, or a patient corresponding to the individual.

The user terminal 200 includes a communication unit, a display unit, a storage unit, and a processor.

The communication unit of the user terminal 200 is configured to communicate with an external system. The communication unit is connected to the radiation sensitivity diagnosis kit 100 using wired/wireless communication to transmit various data associated with radiation sensitivity. Specifically, the communication unit receives a diagnosis result associated with radiation sensitivity of an individual from the radiation sensitivity diagnosis kit 100, for example, the degree of radiation sensitivity for an individual (good, fair, and poor), or whether a therapeutic response is positive or negative, and further, a radiation efficacy diagnostic plot indicating the degree of radiation sensitivity in a plot.

The display unit of the user terminal 200 displays various interface screens for displaying a diagnosis result associated with the radiation sensitivity of the individual. For example, the display unit provides the degree of radiation sensitivity for an individual (good, fair, and poor), or whether a therapeutic response is positive or negative, and further, a radiation efficacy diagnostic plot indicating the degree of radiation sensitivity in a plot.

In various embodiments, the display unit includes a touch screen, and receives, for example, touch, gesture, access, drag, swipe or hovering input performed by an electronic pen or by a portion of the body of the user.

Of course, it is possible to form a new embodiment by combining one or more embodiments among various embodiments of the present invention.

The exemplary embodiments of the present invention have been described in more detail with reference to the accompanying drawings, but the present invention is not essentially limited to the exemplary embodiments, and may be variously modified and carried out within the scope of the technical spirit of the present invention. Accordingly, the various exemplary embodiments disclosed herein are not intended to limit the technical spirit but describe with the true scope and spirit being indicated by the following claims. The scope of the present invention should be construed based on the following appended claims and it should be construed that the technical spirit included within the scope equivalent to the claims belongs to the present invention.

(Explanation of Reference Numerals)

1000: radiation sensitivity diagnosis system 100: radiation sensitivity diagnosis kit
110: storage unit 120: processor
130: communication unit 140: radiation irradiating device
141: CRS plate 143: radiation control unit
145: radiation generating unit 147: imaging device
200: user terminal 300: database providing server
1041: cancer organoid 1042: culture medium
422: input module 424: analysis module
426: output module 428: analysis result
432: reference data

**Industrial Applicability**

Therefore, according to the present invention, the radiation sensitivity diagnosis method can effectively diagnose a patient's radiation sensitivity to cancer cells in order to predict effects of radiation therapy. Therefore, the radiation sensitivity diagnosis method according to the present invention allows a user to accurately and effectively determine whether to perform radiation therapy with respect to a cancer, such as head and neck cancer, lung cancer, esophagus cancer, rectal cancer, anal cancer, cervical cancer, and the like which are suitable for the preservation treatment.

Moreover, the radiation sensitivity diagnosis method according to the present invention can accurately determine radiation sensitivity of individual patients using the most effective factors among various factors previously studied by considering radiation response factors and cell growth factors at the same time.

Furthermore, the radiation sensitivity diagnosis method according to the present invention can obtain data necessary for radiation sensitivity diagnosis by overcoming restrictions, such as a small number of samples and limited time, using characteristic sensitivity diagnosis kit having different total radiation doses irradiated to a plurality of cancer organoids according to a change in relative locations of the plate unit and the radiation shield.

The effects according to the present invention are not limited by the teachings illustrated above, and more various effects are included in this specification.

**Claims**

1. A radiation sensitivity diagnosis method using a radiation sensitivity diagnosis kit including a radiation irradiating device and a control unit, the method comprising:

   (a) dispensing at least one cancer cells separated from an individual on a CRS plate of the diagnosis kit;
   (b) culturing the at least one cancer cell to form one or more cancer organoids by filling the CRS plate with culture media or immersing the CRS plate in a well filled with culture media;
   (c) irradiating radiation rays, which passed a radiation dose control unit, to the CRS plate from a radiation generating unit of the radiation irradiating device;
   (d) measuring cell viability of the at least one cancer organoid;
   (e) obtaining cell experiment-based data on the basis of the measured cell viability; and
   (f) diagnosing radiation sensitivity for the individual by the control unit on the basis of the cell experiment-based data.

2. The radiation sensitivity diagnosis method according to claim 1, wherein the cell experiment-based data includes a cell growth factor and a radiation response factor based on the measured cell viability.

3. The radiation sensitivity diagnosis method according to claim 2, wherein the radiation response factor includes at least one among $RT_{50}$, $\%RT_{50}$, $RT_{AUC}$, and radiation sensitivity index, and
   wherein the $RT_{50}$ and $\%RT_{50}$ correspond to a radiation dose when the viability of the cancer cells is reduced to 50%, and the $RT_{AUC}$ corresponds to an area of a cell activation curve according to the radiation dose.

4. The radiation sensitivity diagnosis method according to claim 2, wherein the cell growth factor is at least one among a cell viability increase rate, a cell growth rate, the degree of cell viability, and a cell size increase rate.

5. The radiation sensitivity diagnosis method according to claim 1, wherein in step (f), the control unit diagnoses the radiation sensitivity using a sensitivity diagnosis model including a sensitivity feature extracted using the radiation response factor and the cell growth factor in the cell experiment-based data.

6. The radiation sensitivity diagnosis method according to claim 5, wherein the sensitivity feature is a radiation sensitivity baseline created by performing curve fitting with respect to the cell experiment-based data including the radiation response factor and the cell growth factor.

7. The radiation sensitivity diagnosis method according to claim 6, wherein the sensitivity diagnosis model calculates a radiation sensitivity index based on the shortest distance between the radiation sensitivity baseline and the new data, which is the cell experiment-based data of the individual.

8. The radiation sensitivity diagnosis method according to claim 6, wherein the sensitivity diagnosis model calculates a radiation sensitivity index based on a distance between a reference point and the new data, which is the cell experiment-based data of the individual.

9. The radiation sensitivity diagnosis method according to claim 5, wherein the radiation diagnosis model is learned to further receive reference data, which is cell experiment-based data of another individual, and extract the sensitivity feature.

10. The radiation sensitivity diagnosis method according to claim 1, wherein the step (d) comprises the steps of:

measuring a cell viability initial value of the at least one cancer organoid;
having additional culturing time of five to seven days; and
measuring a result value of cell viability during the culturing time.

11. The radiation sensitivity diagnosis method according to claim 1, wherein in step (c), the radiation dose control unit irradiates radiation rays with different radiation doses to the at least one cancer organoid.

12. The radiation sensitivity diagnosis method according to claim 11, wherein the radiation dose control unit comprises: a slit type radiation dose modulator for controlling the radiation doses through a slit relatively moving with respect to the CRS plate; a shield movable type radiation dose modulator for controlling radiation irradiation time for each cancer organoid by moving in a rectilinear motion; a density-adjustable radiation shield configured to selectively shield radiation rays by radiation transmitting shield plates which vary a radiation transmission rate with respect to a specific area; and a laminated radiation shield varying the number of the radiation transmitting shield plates according to the radiation doses to be adjusted.

# FIG. 1

| | |
|---|---|
| Dispense cancer cells separated from an individual on a CRS plate of a diagnosis kit | ─ S111 |
| Culture the cancer cells by filling the CRS plate with a culture medium to form organoids | ─ S112 |
| Irradiate radiation rays by a radiation dose control device of the diagnosis kit | ─ S120 |
| Measure cell viability of the cancer organoids to which radiation rays are irradiated | ─ S130 |
| Calculate a cell growth factor and a radiation response factor of the cancer organoids to which radiation rays are irradiated | ─ S140 |
| Diagnose radiation sensitivity using a radiation sensitivity prediction model | ─ S150 |

FIG. 2

**100**

145

140

143

141

1041

147

150

110 — Storage unit

120 — Processor

130 — Communication unit

135 — Radiation Diagnosis module

EP 4 439 063 A1

FIG. 3A

(a)    (b)    (c)    (d)

# FIG. 3B

# FIG. 4

(a)

145
243 { 243-1
       243-2
141
1041a 1041c
1041b
D2

(b)

145
243-1
243-2
141
1041a 1041c
1041b
D2

(c)

145
243-1
243-2
141
1041a 1041c
1041b
D2

# FIG. 5

145
343 { 343-1
       343-2
8[Gy]
341a
1041
141 { 1042
341b
147
0[Gy] 4[Gy] 8[Gy]

# FIG. 6A

(a)             (b)             (c)             (d)

FIG. 6B

# FIG. 6C

443-3       (a)       (b)       443-1       (c)       141    443-2       (d)

# FIG. 7

# FIG. 8

| | |
|---|---|
| Detect an initial value of cell viability | S131 |
| Have additional culturing time of 5 to 7 days | S133 |
| Detect a result value of cell viability during the culturing time | S135 |

FIG. 9

# FIG. 10

(a)

(b)

# FIG. 11

# FIG. 12

# FIG. 13

# FIG. 14A

Shortest distance with
the predicted division line

Radiation sensitivity index [%]

$$= \frac{\text{Shortest distance}}{100\sqrt{2}/2} * 100$$

Therapeutic response negative

Cell growth factor [%]

Therapeutic response positive

Radiation response factor [%]

# FIG. 14B

Distance with
reference point

Radiation sensitivity index

$$= \sqrt{\left(\begin{array}{c}\text{Drug reaction}\\\text{factor}\end{array}\right)^2 + \left(\begin{array}{c}\text{Cell growth}\\\text{factor}\end{array}\right)^2}$$

Therapeutic response
negative

Cell growth factor [%]

Drug reaction factor [%]

Therapeutic response
positive

# FIG. 15A

Radiation sensitivity is low

| CASENUMBER | #1 | #2 | #3 | #4 | #5 | #6 | #7 | #8 | #9 | #10 | #11 | #12 | #13 | #14 | #15 | #16 | #17 | #18 | #19 | #20 | #21 | #22 | #23 | #24 | #25 | #26 | #27 | #28 | #29 | #30 | #31 | #32 | #33 | #34 | #35 | #36 | #37 |
|---|---|---|---|---|---|---|---|---|---|---|---|---|---|---|---|---|---|---|---|---|---|---|---|---|---|---|---|---|---|---|---|---|---|---|---|---|---|
| Radiation sensitivity index [%] | 2.3 | 3.1 | 3.1 | 3.2 | 3.2 | 3.3 | 3.5 | 3.7 | 3.9 | 3.9 | 4.0 | 4.3 | 4.3 | 4.4 | 4.6 | 4.7 | 4.7 | 4.8 | 4.8 | 4.9 | 5.0 | 5.2 | 5.2 | 5.3 | 5.4 | 5.5 | 5.6 | 6.0 | 6.0 | 6.0 | 6.3 | 6.5 | 6.6 | 7.9 | 9.4 | 7.0 | 42.0 |
| Clinical result 1 | | | | | | | | | | | | | | | | | | | | | | | | | | | | | | | | | | | | | |
| Clinical result 2 | | | | | | | | | | | | | | | | | | | | | | | | | | | | | | | | | | | | | |
| Clinical result 3 | | | | | | | | | | | | | | | | | | | | | | | | | | | | | | | | | | | | | |
| Clinical result 4 | | | | | | | | | | | | | | | | | | | | | | | | | | | | | | | | | | | | | |
| Clinical result 5 | | | | | | | | | | | | | | | | | | | | | | | | | | | | | | | | | | | | | |
| Clinical result 6 | | | | | | | | | | | | | | | | | | | | | | | | | | | | | | | | | | | | | |
| Clinical result 7 | | | | | | | | | | | | | | | | | | | | | | | | | | | | | | | | | | | | | |
| Clinical result 8 | | | | | | | | | | | | | | | | | | | | | | | | | | | | | | | | | | | | | |

# FIG. 15B

# FIG. 16

## INTERNATIONAL SEARCH REPORT

| International application No. |
|---|
| **PCT/KR2022/010503** |

**A. CLASSIFICATION OF SUBJECT MATTER**

G01N 33/50(2006.01)i

According to International Patent Classification (IPC) or to both national classification and IPC

**B. FIELDS SEARCHED**

Minimum documentation searched (classification system followed by classification symbols)

G01N 33/50(2006.01); A61N 5/00(2006.01); C12Q 1/6886(2018.01); G01N 33/15(2006.01); G21K 1/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 방사선 조사 장치(irradiation device), 제어부(control element), 방사선 감수성 (radiation sensitivity), 플레이트(plate), 세포 활성도(cell activity), 방사선 반응인자(radiation response factor), 세포 성장인 자(cell growth factor)

**C. DOCUMENTS CONSIDERED TO BE RELEVANT**

| Category* | Citation of document, with indication, where appropriate, of the relevant passages | Relevant to claim No. |
|---|---|---|
| Y | BOSE, M. V. et al. Assessment of the radiation sensitivity of cervical cancer cell lines. Cervical Cancer: Methods and Protocols, Methods in Molecular Biology. 2015, vol. 1249, pp. 351-362.<br>See pages 355-357. | 1-12 |
| Y | KR 10-2014-0039353 A (KOREA UNIVERSITY RESEARCH AND BUSINESS FOUNDATION) 02 April 2014 (2014-04-02)<br>See claims 1 and 9-10; and figure 2. | 1-12 |
| Y | PASCH, C. A. et al. Patient-derived cancer organoid cultures to predict sensitivity to chemotherapy and radiation. Clinical Cancer Research. 01 September 2019, vol. 25, no. 17, pp. 5376-5387.<br>See abstract; page 5378 and page 5381, left column; and figures 3-5. | 2-9 |
| Y | YAO, Y. et al. Patient-derived organoids predict chemoradiation responses of locally advanced rectal cancer. Cell Stem Cell. 02 January 2020, vol. 26, pp. 17-26.<br>See abstract; pages 20-24; and figures 3-4. | 2-9 |
| A | KR 10-2020-0081296 A (NEXT&BIO INC.) 07 July 2020 (2020-07-07)<br>See entire document. | 1-12 |

| ☑ Further documents are listed in the continuation of Box C. | ☑ See patent family annex. |
|---|---|

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 November 2022** | **18 November 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

# EP 4 439 063 A1

**INTERNATIONAL SEARCH REPORT**

| International application No. |
| --- |
| **PCT/KR2022/010503** |

**C.    DOCUMENTS CONSIDERED TO BE RELEVANT**

| Category* | Citation of document, with indication, where appropriate, of the relevant passages | Relevant to claim No. |
| --- | --- | --- |
| A | KR 10-2013-0024904 A (OSAKA PREFECTURAL HOSPITAL ORGANIZATION et al.) 08 March 2013 (2013-03-08)<br>See entire document. | 1-12 |

INTERNATIONAL SEARCH REPORT
Information on patent family members

International application No.

**PCT/KR2022/010503**

| Patent document cited in search report | | | Publication date (day/month/year) | Patent family member(s) | | | Publication date (day/month/year) |
|---|---|---|---|---|---|---|---|
| KR | 10-2014-0039353 | A | 02 April 2014 | KR | 10-1424112 | B1 | 06 August 2014 |
| KR | 10-2020-0081296 | A | 07 July 2020 | KR | 10-2237425 | B1 | 08 April 2021 |
| KR | 10-2013-0024904 | A | 08 March 2013 | IL | 223248 | A | 03 February 2013 |
| | | | | JP | 2011-147434 | A | 04 August 2011 |
| | | | | JP | 5809782 | B2 | 11 November 2015 |
| | | | | WO | 2011-149013 | A1 | 01 December 2011 |

Form PCT/ISA/210 (patent family annex) (July 2022)